# EUROPEAN PATENT APPLICATION

(11) **EP 4 176 834 A1**
(43) Date of publication of application: **10.05.2023**
(21) Application number: 22205250.8
(22) Date of filing: 03.11.2022
(51) Int. Cl.: A61B 34/35, A61B 34/00, A61B 17/00

(54) **REMOTE CONTROL OF ARTICULATED CATHETER EMULATING HAND-HELD MOTIONS**

(30) Priority: 05.11.2021 US 202117520333
(71) Applicant: Covidien LP, Mansfield, MA 02048 (US)
(72) Inventor: KOMP, John W., Dillon, CO 80301 (US)
(74) Representative: Maschio, Antonio

(57) **Abstract**

A system for performing a surgical procedure includes an endoscope. The endoscope includes a catheter attachment and a control handle selectively coupled to a portion of the catheter attachment such that inputs received by the control handle effectuate corresponding reactions of a portion of the catheter attachment when the control handle is coupled to the catheter attachment and when the control handle is separated from the catheter attachment.

## Description

### BACKGROUND

### Technical Field

The present disclosure relates to the field of surgical devices, and in particular, to remotely controlled catheters that emulate hand-held motions.

### Description of Related Art

Minimally invasive surgical procedures have become increasingly prevalent over traditional open surgical techniques. Handheld endoscopes enable clinicians to explore internal cavities of a patient without the need for extensive incisions, reducing patient recovery time and patient discomfort. Endoscopes have been increasingly used to explore narrow luminal networks of the patient, such as the luminal network of the lungs, amongst others. Typical endoscopic procedures involve manual manipulation of the catheter and manual articulation of a distal end portion of the catheter to navigate the various bifurcations and/or undulating nature of the luminal network more easily.

Advances in technology has enabled clinicians to utilize robotic surgical systems to perform various surgical procedures. As can be appreciated, robotic surgical systems enable fine motor control and a steadier grasp on the endoscope. However, much is lost in the way of feedback to the user when using a robotic surgical system over manual manipulation of the endoscope. As such, clinicians lose tactile sensations when utilizing robotic surgical systems, which make it difficult for a clinician to identify how much pressure is being applied to the surrounding tissue when advancing or otherwise manipulating the endoscope within the luminal network. Such difficulties arise during initial insertion of the endoscope within the luminal network, where a clinician may wish to have manual control and tactile feedback from the endoscope, whereas the finer control and steadier hand of the robotic surgical system is preferred as the distal portion of the catheter approaches the surgical site and/or target tissue.

Additionally, the camera view at the distal end of the endoscope reacts differently when the endoscope is manipulated by the robotic surgical system as compared to manual manipulation, as the clinician is permitted to physically rotate the catheter whereas robotic surgical systems are rotationally fixed. Therefore, the view presented to the clinician is not what is expected, leading to difficulty in identifying the direction in which the distal end portion of the catheter must be articulated to traverse the various lumens of the luminal network.

### SUMMARY

In accordance with the present disclosure, a system for performing a surgical procedure includes an endoscope including a catheter attachment and a control handle selectively coupled to a portion of the catheter attachment such that inputs received by the control handle effectuate corresponding reactions of a portion of the catheter attachment when the control handle is coupled to the catheter attachment and when the control handle is separated from the catheter attachment.

In aspects, the catheter attachment may include a catheter housing and a catheter operably coupled to, and extending distally from, a portion of the catheter housing.

In other aspects, the catheter housing may include a drive mechanism that is disposed within a portion of the catheter housing and is configured to effectuate articulation of a distal portion of the catheter.

In certain aspects, the drive mechanism may include a motor, wherein the motor causes the drive mechanism to effectuate articulation of the distal portion of the catheter.

In other aspects, the control handle may include a motor disposed therein, wherein the motor is operably coupled to the drive mechanism when the catheter housing is coupled to the control handle.

In aspects, the system may include a robotic surgical system, a portion of the robotic surgical system configured to be selectively coupled to a portion of the catheter housing when the catheter housing is separated from the control handle.

In other aspects, the catheter housing may include a drive mechanism disposed therein, the drive mechanism selectively couplable to a portion of the robotic surgical system such that when the catheter housing is coupled to the robotic surgical system, the robotic surgical system is operably coupled to the drive mechanism.

In aspects, the control handle may be in selective communication with the robotic surgical system, wherein inputs received on the control handle are received by the robotic surgical system to cause the robotic surgical system to effectuate a corresponding reaction of the catheter.

In other aspects, the control handle may include an inertial measurement unit, wherein movement of the control handle is measured by the inertial measurement unit to effectuate a corresponding reaction of the catheter.

In certain aspects, the control handle may include a control pad, wherein inputs received on the control pad cause the robotic surgical system to effectuate a corresponding reaction of the catheter.

In accordance with another aspect of the present disclosure, a method for performing a surgical procedure includes navigating a distal portion of a catheter attachment within a body cavity of a patient using a control handle selectively coupled to a portion of the catheter attachment, separating the control handle from the catheter attachment, coupling the catheter attachment to a portion of a robotic surgical system, and navigating the distal portion of the catheter attachment to a surgical site using the control handle to remotely control movement of the catheter attachment via the robotic surgical system.

In aspects, navigating a distal portion of the catheter attachment may include articulating a distal portion of a catheter operably coupled to a portion of the catheter attachment using a drive mechanism disposed within a portion of the catheter attachment.

In certain aspects, navigating a distal portion of the catheter may include articulating a distal portion of the catheter using a motor operably coupled to the drive mechanism and disposed within a portion of the catheter attachment.

In other aspects, navigating a distal portion of the catheter may include articulating the distal portion of the catheter using a motor operably coupled to the drive mechanism and disposed within a portion of the control handle.

In certain aspects, coupling the catheter attachment to a portion of the robotic surgical system may include coupling a motor disposed within a portion of the robotic surgical system to the drive mechanism of the catheter attachment.

In accordance with yet another aspects of the present disclosure, a system for performing surgery includes a catheter housing including a catheter operably coupled to, and distally extending from, a portion of the catheter housing, a control handle selectively coupled to a portion of the catheter housing and configured to receive inputs to effectuate reactions of the catheter of the catheter housing, and a robotic surgical system, apportion of the robotic surgical system configured to be operably coupled to the catheter housing when the catheter housing is separated from the control handle.

In aspects, the catheter housing may include a drive mechanism, the drive mechanism disposed within a portion of the catheter housing and configured to effectuate articulation of a distal portion of the catheter.

In other aspects, the control handle may include a motor, the motor disposed within a portion of the control handle and configured to be operably coupled to the drive mechanism when the catheter housing is coupled to the control handle.

In certain aspects, the robotic surgical system may include a motor, the motor disposed within a portion of the robotic surgical system and configured to be operably coupled to the drive mechanism when the catheter housing is coupled to the robotic surgical system.

In aspects, the control handle may be configured to directly control articulation of the distal portion of the catheter when the catheter housing is coupled to the control handle and is configured to remotely control articulation of the distal portion of the catheter when the catheter housing is coupled to the robotic surgical system.

### BRIEF DESCRIPTION OF THE DRAWINGS

Various aspects and embodiments of the disclosure are described hereinbelow with references to the drawings, wherein:
FIG. 1 is a schematic view of a surgical system provided in accordance with the present disclosure;
FIG. 2 is a schematic view of a workstation of the surgical system of FIG. 1;
FIG. 3 is an elevation view of an endoscope provided in accordance with the present disclosure;
FIG. 4 is a rear, perspective view of a catheter housing of the endoscope of FIG. 3;
FIG. 5 is a schematic view of a distal portion of the catheter of the catheter housing of FIG. 4, shown with the distal portion of the catheter articulating in a left and right direction;
FIG. 6 is an exploded view of a drive mechanism of the catheter housing of FIG. 4;
FIG. 7 is a rear, perspective view of a proximal portion of the catheter housing of FIG. 4;
FIG. 8 is an elevation view of a control handle of the endoscope of FIG. 3;
FIG. 9 is a rear, perspective view of the control handle of FIG. 8;
FIG. 10 is a plan view of the control handle of FIG. 8;
FIG. 11 is an elevation view of the control handle of FIG. 8, showing a trigger associated therewith;
FIG. 12 is an enlarged view of the area of detail indicated in FIG. 10;
FIG. 13 is a perspective view of a robotic surgical system of the surgical system of FIG. 1;
FIG. 14 is a perspective view of a distal portion of the catheter of the catheter housing of FIG. 4;
FIG. 15 is a perspective view of the catheter of the catheter housing of FIG. 4 advanced within a body cavity of a patient; and
FIG. 16 is a flow diagram of a method of performing a surgical procedure provided in accordance with the present disclosure.

### DETAILED DESCRIPTION

The present disclosure is directed to a surgical system having a workstation operably coupled to an endoscope, which in turn, is selectively couplable to a robotic surgical system. The endoscope is formed from two sections; a catheter housing and a control handle. The catheter housing and the control handle are configured to be selectively coupled to one another, such that the catheter housing may be controlled either manually, when the control handle is coupled thereto, or remotely via the robotic surgical system, when the catheter housing is coupled to the robotic surgical system.

The catheter housing includes a recess formed in an outer surface thereof adjacent a proximal portion that is configured to mate to a corresponding recess formed in an outer surface of a distal portion of the control handle and to a cradle of the robotic surgical system. In this manner, the recesses of each of the catheter housing and the control handle enable the catheter housing to be releasably engaged to the control housing. The catheter housing includes a catheter operably coupled thereto which terminates at a distal end portion having a camera or any other suitable sensor or sensors or device or devices disposed therein. To manipulate the catheter, the catheter housing includes a drive mechanism disposed therein which may include one or more pull wires to control articulation of the distal portion of the catheter in a left, right, up, or down direction, or combinations thereof. In embodiments, the drive mechanism may include one or more motors which when actuated, effectuate movement of the pull wires and rotation of the catheter relative to the catheter housing. It is envisioned that the drive mechanism may not include a motor, and rather, motors may be disposed in the control handle and a portion of the robotic surgical system. In this manner, the catheter housing may be disposable whereas the control handle may be reusable.

The control handle includes a user interface disposed on an outer surface thereof which enables a user to control articulation of the distal portion of the catheter in a single plane *(e.g.,* left and right) when the catheter housing is coupled to the control handle, and enables a user to control articulation of the distal portion of the catheter in two planes *(e.g.,* left, right, up, and down) when the catheter housing is coupled to the cradle of the robotic surgical system. As can be appreciated, when the catheter housing is coupled to the control handle, rotation of the catheter is effectuated by manually rotating the control handle, and thereby the catheter. When the catheter housing is coupled to the cradle of the robotic surgical system, the catheter is rotationally fixed, thereby inhibiting rotation of the catheter relative to the cradle.

As can be appreciated, when the catheter housing is coupled to the cradle of the robotic surgical system and the distal portion of the catheter is articulated in a 180-degree hemisphere *(e.g.,* two planes), the view captured by the camera and displayed to the user no longer corresponds to what the user was expecting. For example, when the user rotates their hand to effectuate rotation of the catheter housing when the catheter housing is coupled to the control handle, what the user expects to be up or down corresponds to up or down on the display. However, when the catheter housing is coupled to the cradle of the robotic surgical system, the catheter is inhibited from rotating and therefore, the view captured by the camera and displayed to the user is rotated from what the user would expect. An application associated with the workstation interprets the data received from the catheter (*e.g*., orientation, position, image data, etc.) and automatically rotates the view before being displayed to the user. In this manner, the view displayed to the user corresponds to the view in which the user was expecting. These and other aspects of the present disclosure are described in further detail herein.

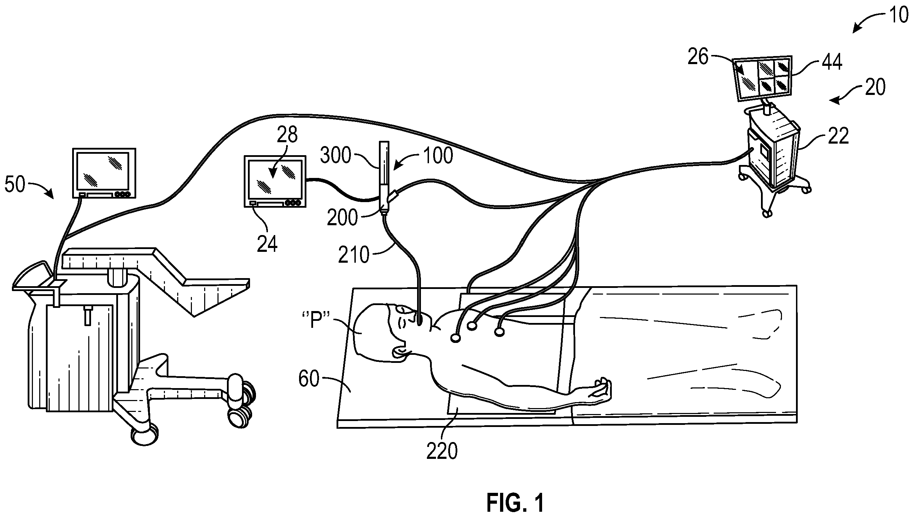

Turning now to the drawings, FIG. 1 illustrates a surgical system provided in accordance with the present disclosure and generally identified by reference numeral 10. The surgical system includes an endoscope 100, a workstation 20 operably coupled to the endoscope 100, and a robotic surgical system 50 operably coupled to the workstation 20 and in selective communication with the endoscope 100. The patient is shown lying on an operating table 60 with the endoscope 100 inserted through the patient's mouth and into the patient's airways, although it is contemplated that the endoscope 100 may be inserted into any suitable body cavity of the patient, depending upon the procedure being performed.

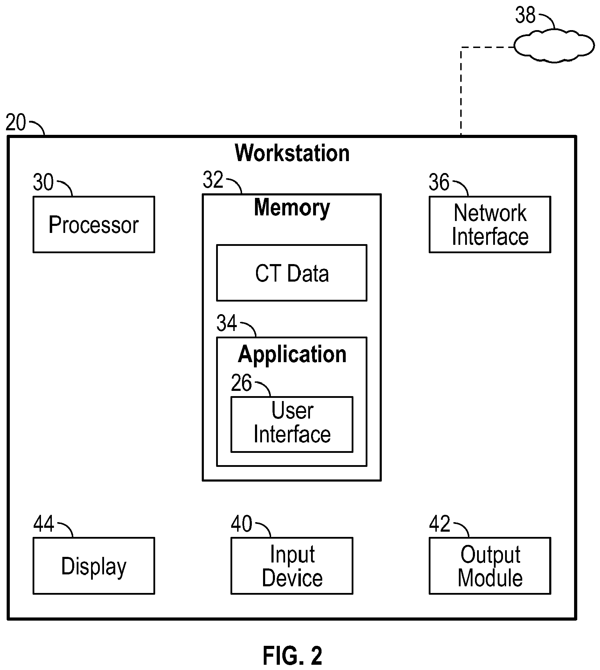

Continuing with FIG. 1 and with additional reference to FIG. 2, the workstation 20 includes a computer 22 and a display 24 that is configured to display one or more user interfaces 26 and 28. The workstation 20 may be a desktop computer or a tower configuration with the display 24 or may be a laptop computer or other computing device. The workstation 20 includes a processor 30 which executes software stored in a memory 32. The memory 32 may store video or other imaging data captured by the endoscope 100 or pre-procedure images from, for example, a computer-tomography (CT) scan, Positron emission tomography (PET), Magnetic Resonance Imaging (MRI), Cone-beam CT, amongst others. In addition, the memory 32 may store one or more applications 34 to be executed on the processor 30. Though not explicitly illustrated, the display 24 may be incorporated into a head mounted display such as an augmented reality (AR) headset such as the HoloLens offered by Microsoft Corp.

A network interface 36 enables the workstation 20 to communicate with a variety of other devices and systems via the Internet. The network interface 36 may connect the workstation 20 to the Internet via a wired or wireless connection. Additionally, or alternatively, the communication may be via an ad-hoc Bluetooth^{®} or wireless networks enabling communication with a wide-area network (WAN) and/or local area network (LAN). The network interface 36 may connect to the Internet via one or more gateways, routers, and network address translation (NAT) devices. The network interface 36 may communicate with a cloud storage system 38, in which further image data and videos may be stored. The cloud storage system 38 may be remote from or on the premises of the hospital such as in a control or hospital information technology room. An input device 40 receives inputs from an input device such as a keyboard, a mouse, voice commands, amongst others. An output module 42 connects the processor 30 and the memory 32 to a variety of output devices such as the display 24. In embodiments, the workstation 20 may include its own display 44, which may be a touchscreen display.

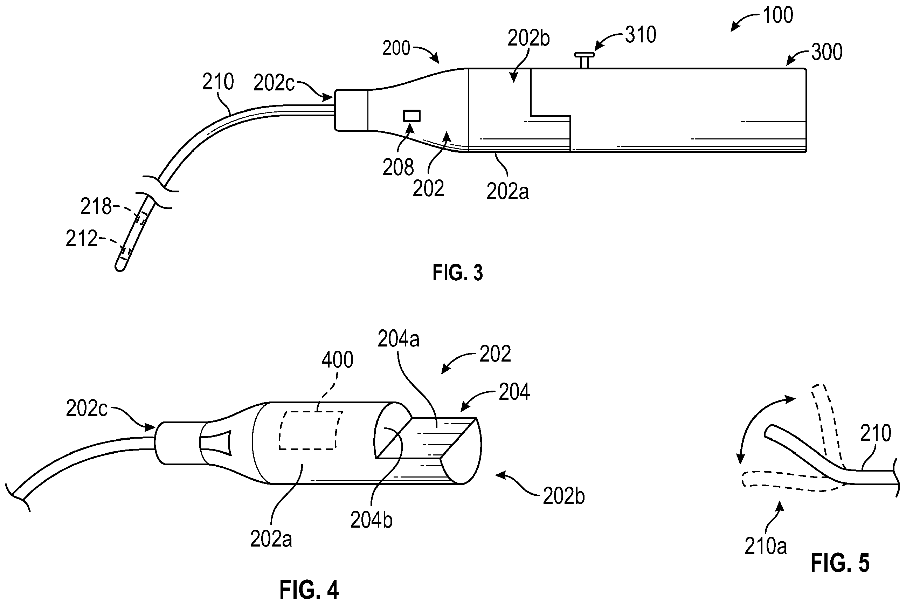

With reference to FIGS. 3-7, the endoscope 100 includes a handle portion 102 that is formed from a catheter attachment 200 and a control handle 300 that is selectively couplable to the catheter attachment 200. The catheter attachment 200 includes a catheter housing 202 and a catheter 210 operably coupled to a distal portion of the catheter housing 202 and extending distally therefrom and terminating at a distal end portion 210a. It is contemplated that the catheter 210 may include a camera 212, ultrasound transponder, photoacoustic imager, etc. disposed at the distal end portion 210a thereof. In this manner, the catheter 210 may capture images, either white light, infrared, ultrasound, photoacoustic, etc. extending axially from the distal end portion 210a of the catheter 210 or in embodiments, in a 360-degree ring about the distal end portion 210a of the catheter 210.

The catheter housing 202 defines a generally cylindrical profile defining an outer surface 202a, which extends between opposed proximal and distal end portions 202b and 202c, respectively. The catheter housing 202 includes an outer dimension that is configured to be operably received within a cradle or other portion of the robotic surgical system 50, such that the catheter 210 may be controlled manually via the handle portion 102 or remotely via the robotic surgical system 50, as will be described in further detail hereinbelow.

The outer surface 202a of the catheter housing 202 defines a recess 204 defined therein adjacent to and extending through the proximal end portion 202b. The recess 204 defines an upper surface 204a and a distal end surface 204b. As will be described in further detail hereinbelow, the recess 204 includes profile that is complementary to a corresponding feature formed on a portion of the control handle 300 and formed on a portion of the robotic surgical system 50, such that the catheter housing 202 may be releasably coupled to the control handle 300 or the robotic surgical system 50. Although generally illustrated as extending halfway through a thickness of the catheter housing 202, it is envisioned that the recess 204 may include any suitable dimension capable of being releasably engaged to the corresponding feature of the control handle 300 or robotic surgical system 50.

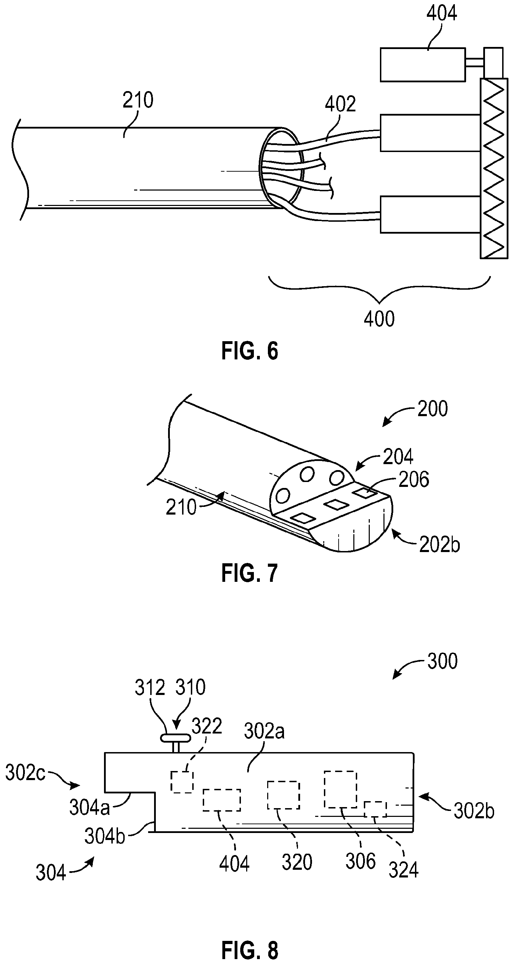

The catheter housing 202 includes a drive mechanism 400 (FIG. 6) disposed within an inner portion thereof that is operably coupled to a proximal portion of the catheter 210. The drive mechanism 400 effectuates manipulation of a distal portion 210a of the catheter 210 in five degrees of freedom *(e.g.,* left, right, up, down, and rotation), although it is contemplated that the drive mechanism may be configured to effectuate movement in greater or fewer degrees of freedom without departing from the scope of the present disclosure. In this manner, the distal portion 210 of the catheter 210 is permitted to articulate through a hemisphere of 180 degrees (*e.g*., two planes of articulation). It is envisioned that the drive mechanism 400 may be a cable actuated using artificial tendons or pull wires 402 *(e.g.,* metallic, non-metallic, composite, etc.) or may be a nitinol wire mechanism. In embodiments, the drive mechanism 400 may include motors 404 or other suitable devices capable of effectuating movement of the pull wires 402. In this manner, the motors 404 are disposed within the catheter housing 202 such that rotation of the motors 404 effectuates a corresponding extension or retraction of the pull wires 402, and therefore, a corresponding articulation of the distal portion 210a of the catheter 210.

The catheter housing 202 includes one or more electrical contacts 206 (FIG. 7) disposed on all or a portion of the proximal end portion 202b, upper surface 204a of the recess 204, or the distal end surface 204b of the recess 204. The electrical contacts 206 are in electrical communication with the camera 212 (FIG. 1), ultrasound transponder, etc. and the motors 404 of the drive mechanism 400. As can be appreciated, the electrical contacts 206 are configured to engage corresponding contacts disposed on a portion of the control handle 300 and/or robotic surgical system 50 such that inputs received by the control handle 300 effectuate corresponding actions of the motors 404, camera 212, etc. Additionally, the electrical contacts 206 may transmit data from the motors 404, camera 212, etc. to the control handle 300, which ultimately transmits the data to the workstation 20, as will be described in further detail hereinbelow. In embodiments, the catheter housing 202 may include electrical connectors (*e.g*., plug and socket connectors, amongst others), or may include a combination of electrical connectors and electrical contacts 206 without departing from the scope of the present disclosure.

With reference to FIGS. 8-12, the control handle 300 defines a generally cylindrical profile defining an outer surface 302a, which extends between opposed proximal and distal end portions 302b and 302c, respectively. The control handle 302 includes an outer dimension that is generally similar to the outer dimension of the catheter housing 202, although it is contemplated that the outer dimension of the control handle 300 may be any suitable dimension capable of being easily grasped by a user and may be the same or different than that of the catheter housing 202. Similarly, it is envisioned that the outer profile of the control handle may include any suitable profile for grasping by a user and may be the same or different than the outer profile of the catheter housing 202. The outer surface 302a of the control handle 300 defines a recess 304 therein adjacent to and extending through the distal end portion 302c. The recess 304 defines a lower surface 304a and a proximal end surface 304b. As can be appreciated, the recess 304 defines a profile that corresponds substantially to the profile of the recess 204 of the catheter housing 202 such that the catheter housing 202 and the control handle 300 may interlock or otherwise have the upper surface 204a of the catheter housing 202 be disposed adjacent to the lower surface 304a of the control handle 300 when the catheter housing 202 and the control handle 300 are placed in an interlocked condition. In this manner, it is contemplated that the catheter housing 202 and the control housing 300 may include an interlocking feature, such as detents, resilient fingers, magnetic latches, amongst others to selectively couple the catheter housing 202 to the control handle 300. In one non-limiting embodiment, one of the catheter housing 202 or the control handle 300 may include a socket *(e.g.,* cavity) and the remaining one of the catheter housing 202 or the control handle 300 may include a plug *(e.g.,* boss) such that the socket receives the plug and releasably coupled the catheter housing 202 to the control handle 300. Although generally described has having recesses, it is contemplated that the catheter housing 202 and the control handle 300 may not include recesses 204 and 304, respectively, and rather, the catheter housing 202 may be selectively coupled to the control handle 300 using any suitable means, such as clips, fasteners, resilient fingers, amongst others.

The lower surface 304a of the recess 304 include one or more electrical contacts 306 disposed thereon. As can be appreciated, the number and location of the electrical contacts 306 may correspond to the number and location of the electrical contacts 206 disposed on the upper surface 204a of the recess 204 of the catheter housing 202. In this manner, the electrical contacts 206 and the electrical contacts 306 cooperate to transmit inputs from the control handle 300 and/or the robotic surgical system 50 to the motors 404, camera 212, etc. associated with the catheter housing 202. As can be appreciated, the control handle 300 includes a corresponding type of electrical connection to that of the catheter housing 202, such that if the catheter housing 202 include electrical connectors, the control handle 300 includes corresponding electrical connectors, etc. such that electrical energy may freely flow between each of the catheter housing 202 and the control handle 300 for both control of the catheter 201 and/or powering the motors, sensors, etc. via an energy storage device (not shown). It is envisioned that the energy storage device may be disposed in the control handle 300, or in embodiments, may be disposed in the catheter housing 202 and may be charged wirelessly, via a physical cable, or a charging dock or the like, etc.

In embodiments, the catheter housing 202 may communicate with the control handle 300 wirelessly via Wi-Fi, Bluetooth^{®}, amongst others. In this manner, each of the catheter housing 202 and the control handle 300 may include a wireless transmitter and receiver 306 to transmit and/or receive inputs and data therebetween. As can be appreciated, wireless communication obviates the need for electrical contacts or electrical connectors, simplifying the construction of each of the catheter housing 202 and control handle 300, enabling each of the catheter housing 202 or control handle 300 to be reduced in size, and minimizes chances of communication failures, electrical shorts, etc. The control handle 300 communicates with the workstation 20 and/or robotic surgical system 50 to transmit data captured by the camera 212 and various sensors disposed within the catheter 210. It is contemplated that the control handle 300 may be physically coupled to the workstation 20 and/or robotic surgical system 50 via a control cable (not shown), or in embodiments, may communicate with the workstation 20 and/or robotic surgical system 50 wirelessly. In embodiments, the catheter housing 202 may wirelessly communicate with the robotic surgical system 50 to enable transmission of data captured by the camera 212 or other sensors coupled to the catheter 210 or catheter housing 202.

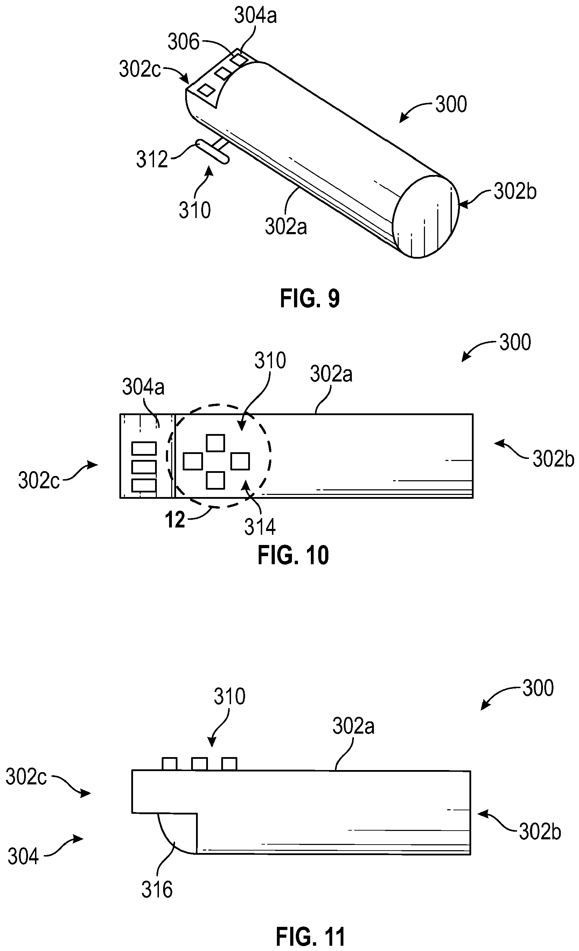

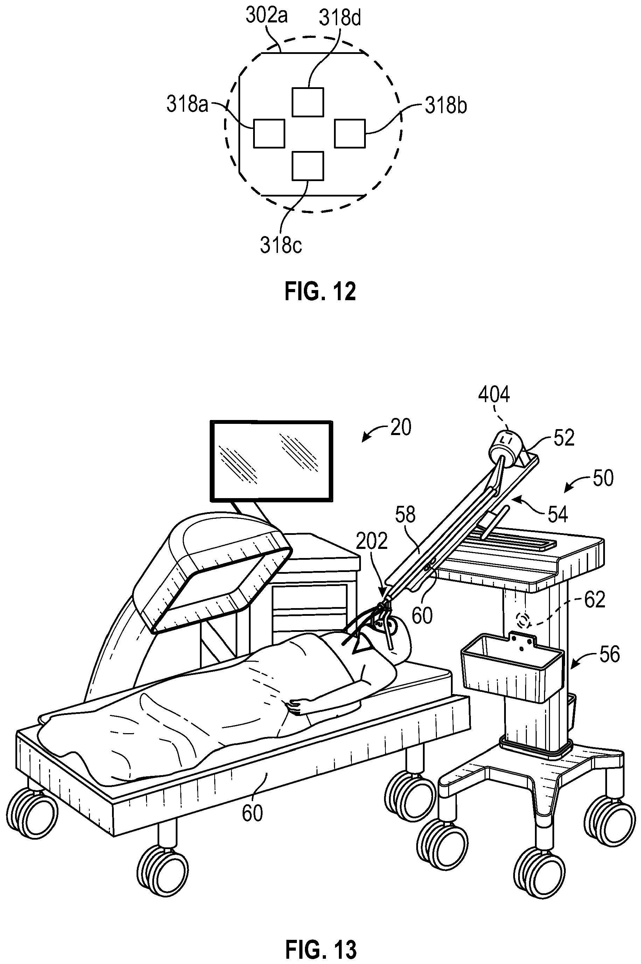

Continuing with FIGS. 8-12, the control handle 300 includes a user interface 310 disposed thereon. The user interface 310 enables a user to control articulation of the distal portion 210a of the catheter 210. The user interface 310 includes a joystick 312 protruding from the outer surface 302a of the control handle 300 that is configured to be manipulated by a user. In embodiments, the joystick 312 may be manipulated by a user's thumb and may be manipulated in two-axes (*e.g*., forward-backward and left-right), although it is contemplated that the joystick 312 may be configured to be manipulated using any suitable means and may be manipulated in any number of axes, and in embodiments, may be manipulated 360-degrees about an axis. It is contemplated that in lieu of a joystick, the user interface 310 may include one or more buttons 314 (FIG. 10) or may include a trigger 316 (FIG. 11) disposed adjacent the recess 304 to effectuate axial (*e.g*., forward and backward) motion when the catheter housing 202 is removed from the control handle 300 and coupled to the robotic surgical system 50. In one non-limiting embodiment, the control handle 300 includes a four button-pad 318 (FIG. 12) disposed thereon having a first button 318a, a second button 318b, a third button 318c, and a fourth button 318d disposed in a cross or plus-sign configuration such that the first and second buttons 318a, 318b are longitudinally aligned on the control handle 300 and the third and fourth buttons 318c, 318d are aligned in a transverse direction to the first and second buttons 318a, 318b. Although generally described as being four separate buttons, it is contemplated that the four-button pad 318 may be a rocker pad or other suitable type of input device without departing from the scope of the present disclosure. As can be appreciated, the first and second buttons 318a, 318b correspond to forward and backward motions whereas the third and fourth buttons 318c, 318d correspond to left and right motions.

In operation, when the control handle 300 is coupled to the catheter housing 202 (*e.g.,* in a handheld mode), the first and second buttons 318a, 318b are disabled such that only the third and fourth buttons 318c, 318d are active to effectuate left and right articulation of the distal end portion 210a of the catheter 210 *(e.g.,* a single plane of motion). When the control handle 300 is decoupled from the catheter housing 202 and the catheter housing 202 is coupled to the robotic surgical system 50, all four of the first, second, third, and fourth buttons 318a, 318b, 318c, 318d are active such that the first and second buttons 318a, 318b effectuate axial movement of the catheter 210 in and out, respectively, and the third and fourth buttons 318c, 318d effectuate left and right articulation of the distal end portion 210a of the catheter 210, respectively (*e.g*., two planes of motion). In this manner, the user interface 310 controls articulation of the distal end portion 210a of the catheter 210 at all times (*e.g*., when the catheter housing 202 is coupled to the control handle 300 or to the robotic surgical system 50).

As can be appreciated, the first and second buttons 318a, 318b are unnecessary when the catheter housing 202 is coupled to the control handle 300, as movement of the user's hand in the forward and backward directions effectuates corresponding insertion and retraction of the catheter 210. Further, when the catheter housing 202 is coupled to the control handle 300, articulation of the distal end portion 210a of the catheter 210 is limited to a single plane *(e.g.,* left and right) and rotation of the distal end portion 210a of the catheter 210 is effectuated by rotation of the entire handle portion 102. When the catheter housing 202 is coupled to the robotic surgical system 50, the catheter housing 202 is constrained such that rotation of the catheter housing 202 is not permitted. In this manner, two-plane articulation (*e.g*., 180-degree hemisphere) is permitted such that the distal end portion 210a of the catheter 210 can be articulated left, right, up, down, and all areas therebetween in a 180-degree hemisphere.

In embodiments, the control handle 300 may include an inertial measurement unit (IMU) 320 disposed therein to measure movement of the control handle 300 in an axial direction or in a rotational direction (*e.g*., axial motion and rotational motion). The movement detected by the IMU 320 is interpreted by a processor 322 disposed within the control handle 300, which then generates electrical signals to cause articulation of the distal end portion 210a of the catheter 210 or to cause a portion of the robotic surgical system 50 to axially translate the catheter housing 202, and therefore, the catheter 210. In embodiments where the control handle 300 includes a trigger 316, it is envisioned that the trigger 316 may be utilized as a go-no go button to inhibit axial movement of the catheter housing 202 *(e.g.,* when in a first, undepressed position) or permit axial movement of the catheter housing 202 *(e.g.,* when in a second, depressed position). In embodiments, the trigger 316 may be held in the second, depressed position to enable the control handle 300 to be utilized as a three-dimensional (3D) wand, where the IMU 320 senses movement of the control handle 300 in a 3D environment. The IMU 320 measures the movement of the control handle 300 and translates the measurements into two-plane articulation of the distal end portion 210a of the catheter 210 when the catheter housing 202 is coupled to the robotic surgical system 50. In this manner, the catheter 210 remains stationary while the distal end portion 210a is free to move in a 180-degree hemisphere about the axis of the catheter 210. In this manner, the rotational position of the catheter 210 remains stationary while the distal end portion 210a of the catheter 210 is free to rotate about the axis of the catheter 210.

Returning to FIG. 3, the outer surface 202a of the catheter housing 202 may include one or more apertures 208 defined therethrough that is in open communication with a working channel (not shown) defined through a portion of the catheter 210. The aperture 208, and therefore, the working channel, is configured to slidably receive a tool or other suitable device therein to enable a user to perform various operations at the surgical site.

Turning to FIG. 13, the robotic surgical system 50 includes drive mechanism 52 including a robotic arm 54 operably coupled to a base or cart 56. The robotic arm 54 includes a cradle 58 that is configured to receive a portion of the catheter housing 202 thereon. The catheter housing 202 is coupled to the cradle 58 using any suitable means (*e.g*., straps, mechanical fasteners, couplings, amongst others) such that the catheter housing 202 is inhibited from rotating with respect to the cradle 58. The cradle 58 or other suitable portion of the robotic arm 54 includes electrical contacts 60 disposed thereon corresponding to the electrical contacts 206 of the catheter housing 202 such that the robotic surgical system 50 is permitted to communication with the motors 404, camera 212, etc. disposed within the catheter housing 202. As can be appreciated, the orientation of the distal end portion 210a of the catheter 210 may have been rotated from its previous position during the process of placing the catheter housing 202 in the cradle 58, in that the catheter housing 202 may have been rotated by the user when placing the catheter housing 202 in the cradle 58. To alleviate this issued, it is contemplated that the robotic arm 54, the cradle 58, or the electrical contacts 60 can automatically rotate to the rotational position of the distal end portion 210a of the catheter, and therefore, the rotational orientation of the control handle 300 before removal of the control handle 300 from the catheter housing 202 to ensure that the rotational position of the catheter 210 does not inadvertently change when placing the control handle 300 into the cradle 58.

The robotic surgical system 50 includes a wireless communication system 62 disposed therein such that the control handle 300 may wirelessly communicate with the robotic surgical system 50 and/or the workstation 20 via Wi-Fi, Bluetooth^{®}, amongst others. As can be appreciated, the robotic surgical system 50 may omit the electrical contacts 60 altogether and may communicate with the catheter housing 202 wirelessly. In embodiments, the robotic surgical system may utilize both electrical contacts 60 and wireless communication. The wireless communication system 62 is substantially similar to the wireless network interface 36 of the workstation 20, and therefore, the wireless communication system 62 will not be described in detail herein in the interest of brevity. In embodiments, the robotic surgical system 50 and the workstation 20 may be one in the same or may be widely distributed over multiple location within the operating room. It is contemplated that the computer 22 may be disposed in a separate location and the display 44 may be an overhead monitor disposed within the operating room.

The wireless communication system 62 is wirelessly coupled to the wireless transmitter and receiver 306 disposed within the control handle 300 such that inputs on the control handle 300 are wirelessly transmitted to the wireless communication system 62, which in turn, effectuates a corresponding action by either the catheter housing 202 or the cradle 58 of the robotic arm 54. In this manner, actuating the first or second buttons 318a, 318b of the control handle 300 cause the cradle 58 to translate axially forward or backward to insert or retract the catheter 210. Likewise, actuating the second or third buttons 318c, 318d of the control handle 300 cause the robotic surgical system 50 to send signals to the catheter housing 202 to cause the distal end portion 210a of the catheter 210 to articulate left or right. It is envisioned that the wireless communication system 62 may communicate with a point-to-point, mesh network, etc. such that each of the control handle 300, the robotic surgical system 50, the workstation 20, and the catheter housing 202 are connected to the same network.

Although generally described as having the motors 402 disposed within the catheter housing 202, it is contemplated that the catheter housing 202 may not include motors 402 disposed therein (FIG. 8). In this manner, the drive mechanism 400 disposed within the catheter housing 202 may interface with motors 402 disposed within the control handle 300 and the cradle 58 of the robotic surgical system 50. In embodiments, the catheter housing 202 may include a motor or motors 402 for controlling articulation of the distal end portion 210a of the catheter in one plane *(e.g.,* left/null, right/null, etc.) and the drive mechanism 52 of the robotic surgical system 50 may include at least one motor 404 to effectuate the second axis of rotation and for axial motion. In this manner, the motor 402 of the catheter housing 202 and the motors 404 of the robotic surgical system cooperate to effectuate four-way articulation of the distal end portion 210a of the catheter 210 and effectuate rotation of the catheter 210 when the control handle 300 is rotated. As can be appreciated, by removing the motors 402 from the catheter housing 202, the catheter housing 202 becomes increasingly cheaper to manufacture and may be a disposable unit. In this manner, the control handle 300 may contain the expensive components and be reusable, minimizing cleaning time and reducing capital expenditure.

As can be appreciated, the difference in how the distal end portion 210a of the catheter 210 rotates when the catheter housing 202 is coupled to the control handle 300 compared to how the distal end portion 210a of the catheter 210 rotates when the catheter housing 202 is coupled to the cradle 58 of the robotic surgical system 50 may cause the view by which the user navigates the catheter 210 to behave differently. When coupled to the control handle 300, rotation of the control handle 300 effectuates a corresponding rotation of the catheter housing 202 and the catheter 210. In this manner, the view from the camera 212 disposed at the distal end portion 210a of the catheter 210 always remains in line with the orientation of the control handle 300 *(e.g.,* up is always up and down is always down). However, when the catheter housing 202 is coupled to the cradle 58 of the robotic surgical system 50, the catheter housing 202 is inhibited from rotating. Rather, rotation of the control handle 300 effectuates an articulation of the distal end portion 210a of the catheter 210 in a 180-degree hemisphere, while the catheter 210 remains stationary. As can be appreciated, with the catheter 210 remaining stationary and only the distal end portion 210a of the catheter 210 rotating, the view displayed on the display 24 no longer corresponds to what the user is expecting. For example, as the distal end portion 210a of the catheter 210 is rotated, what was up in the displayed view rotates, and therefore, a user input to cause the distal end portion 210a of the catheter 210 to articulate up no longer corresponds to up on the display 24. Therefore, the software application 34 associated with the workstation 20 interprets the data received by the processor 30 to determine the orientation of the distal end portion 210a of the catheter 210 relative to the catheter housing 202. Using this data, the software application 34 causes the displayed view to rotate to or otherwise be manipulated to display a view corresponding to how a user would expect the view to look when rotating the control handle 300 *(e.g.,* as if the catheter 210 itself has been rotated).

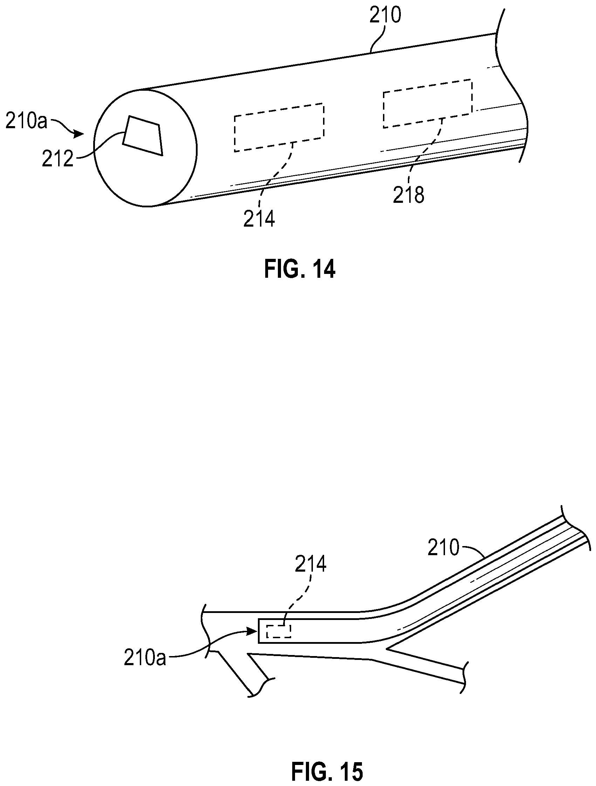

Turning to FIGS. 14 and 15, it is envisioned that the catheter 210 may include a fiber bragg grating 214 disposed therein with an end portion thereof in contact with the distal end portion 210a of the catheter 210. Although generally described as being disposed within the catheter 210, it is contemplated that a portion of the fiber bragg grating 214 may be exposed at the distal end portion 210a of the catheter 210. In this manner, the fiber bragg grating 214 measures a pressure effectuated on the distal end portion 210a of the catheter 210 or upon the fiber bragg grating 214 itself, depending upon the means by which the fiber bragg grating 214 is disposed on the catheter 210. As the catheter 210 is advanced within the patient's body, the fiber bragg grating 214 measures the contact pressure effectuated upon the distal end portion 210a of the catheter 210 and transmits a signal to a haptic feedback generator 324 (FIG. 8) disposed within a portion of the control handle 300. As can be appreciated, when the catheter housing 202 is coupled to the cradle 58 of the robotic surgical system 50, the direct feedback from the distal end portion 210a of the catheter 210 being advanced within the portion of the patient's body is no longer provided, as the control handle 300 is decoupled from the catheter housing 202, and therefore, the catheter 210. As such, the fiber bragg grating 214, and the haptic feedback generator 324, generate feedback for the user to simulate direct control of the catheter 210. In embodiments, the fiber bragg grating 214 may measure a pressure caused by the catheter 210 dilating a vessel, and if so, with how much force (FIG. 15). This measurement is transmitted to the haptic feedback generator 324 of the control handle 300 to denote to the user that the catheter 210 is dilating a vessel and provides feedback as to how much force is being applied to the vessel during dilation. Although generally described as transmitting data to the haptic feedback generator 324, it is contemplated that the sensed data from the fiber bragg grating 214 may be transmitted to the processor 30 of the workstation 20, which in turn, instructs the haptic feedback generator 324 to generate feedback corresponding the to pressure sensed by the fiber bragg grating 214. It is contemplated that the haptic feedback generator 324 may be disabled in certain instances to avoid the generation of feedback when it is not wanted, such as when the control handle 300 is placed on a surface and free from the user's hand. In embodiments, the haptic feedback generator 324 may be toggled on or off using a switch (not shown) or other suitable mechanism.

In embodiments, the catheter 210 includes a location sensor, such as an electromagnetic (EM) sensor 218 (FIG. 3) which receives electromagnetic signals from an electromagnetic field generator 220 (FIG. 1) which generates three or more electromagnetic fields. The application 34 stored in the memory 32 associated with the workstation 20 and/or the robotic surgical system 50 is executed by the processor 30 to determine the position of the EM sensor 218 in the EM field generated by the electromagnetic field generator 220. Although generally described as being an EM sensor, it is contemplated that other position sensors may be utilized, such as ultrasound sensor, flex sensors, fiber Bragg grating (FBG), robotic position detection sensors, amongst others.

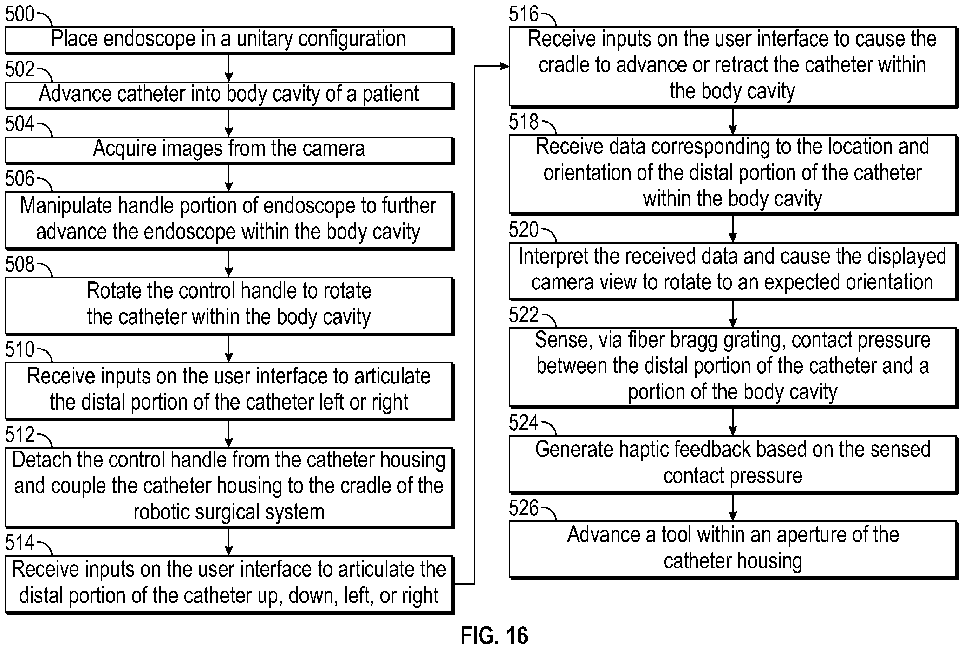

With reference to FIGS. 1-15 and 16, a method of utilizing the surgical system 10 to perform a surgical procedure is illustrated. Initially, in step 500, the endoscope 100 is placed in a unitary configuration having the catheter housing 202 and the control handle 300 coupled to one another. In step 502, the catheter 210 is advanced into a body cavity of a patient. At this point, in step 504, the camera 212 may begin acquiring images, which are then displayed on the display 24 of the workstation 20, and the electromagnetic field generator 220 may begin sensing the location of the EM sensor 218 within the magnetic field and the location of a distal portion of the catheter 210 may be displayed on the display 24. Once inserted within the body cavity of the patient, the catheter 210 is further advanced within the body cavity of the patient in step 506 by manipulating the handle portion 102, which includes the catheter attachment 200 coupled to the control handle 300, such that movement of the control handle 300 in a proximal direction causes the catheter 210 to retract from the body cavity whereas movement of the control handle 300 in a distal direction causes the catheter 210 to advance into the body cavity. As can be appreciated, rotating the control handle 300 in step 508 about a longitudinal axis effectuates a corresponding rotation of the catheter 210 within the body cavity. In step 510, inputs are made on the user interface 310 to cause the distal portion 210a of the catheter 210 to articulate the distal portion 210a in a single plane, either left or right.

As can be appreciated, the clinician is adept at navigating a catheter or endoscope to or near target tissue or a surgical site within a body cavity of a patient. Specifically, the clinician is able to quickly and accurately navigate a liminal network of the patient and advance/retract or articulate the distal portion 210a of the catheter 210. However, as the distal portion 210a of the catheter 210 approaches the target tissue, finer control of the advancement, retraction, and articulation of the catheter is beneficial in order to more accurately place the distal portion 210a of the catheter proximate the target tissue or surgical site. As such, in step 512, the control handle 300 may be detached or otherwise removed from the catheter housing 202 and the catheter housing 202 may be placed within a portion of the cradle 58 of the robotic surgical system 50 such that the catheter housing 202 is operably coupled to the cradle 58. As can be appreciated, once coupled to the cradle 58, rotation of the catheter 210 is constrained and otherwise inhibited. Thus, in step 514, inputs made on the user interface 310 of the control handle 300 effectuate articulation of the distal portion 210a of the catheter 210 in two axes, about a 180-degree hemisphere. Further, in step 516, inputs made on the user interface 310 of the control handle 300 cause the cradle 58 to advance or retract along a longitudinal axis, thereby effectuating advancement or retraction of the distal portion 210a of the catheter 210 in the body cavity of the patient. In embodiments, the inertial measurement unit 320 measures movement of the control handle 300 in a longitudinal direction or a rotational direction, which then causes the distal portion 210a of the catheter 210 to advance or retract or articulate corresponding with the measured movement.

As the distal portion 210a of the catheter 210 is rotated, the images captured by the camera 212 and displayed on the display 24 no longer correspond to the view in which the user was expecting to see. In other words, what the user expects to be an upward motion will no longer correspond to the articulation of the distal portion 210a of the catheter 210 as the distal portion 210a rotates. In this manner, in step 518, the workstation 20 receives data corresponding to the location and orientation of the distal portion 210a of the catheter 210 within the body cavity of the patient. In step 520, the workstation 20 interprets the received data and causes the view presented to the user on the display 24 to correspond to the view the user expects to see. In this manner, the workstation rotates or otherwise repositions the view displayed to the user to correspond to the view the user expects to see. As can be appreciated, by manipulating the view presented to the user on the display 24 as the distal portion 210a of the catheter 210 rotates, the user is not required to retrain or otherwise interpret the view differently when manually operating the endoscope 100 (*e.g*., when the catheter housing 202 is coupled to the control handle 300) and when operating the endoscope 100 remotely (*e.g*., when the catheter housing 202 is coupled to the cradle 58 of the robotic surgical system 50).

As can be appreciated, the use of the robotic surgical system 50 to control movement of the catheter 210 provide finer control over the catheter 210 and enables the clinician to rely on the cradle 58 of the robotic surgical system 50 to maintain the position of the catheter relative to the target tissue or surgical site. In this manner, the clinician may release the control handle 300 and have both hands available to manipulate a tool (not shown) or other device inserted within the aperture 208 of the catheter housing 202. Additionally, if the position of the catheter 210 needs to be adjusted, the clinician can regrasp the control handle 300 to causes the robotic surgical system 500 to advance, retract, or otherwise articulate the catheter 210 to place the distal portion 210a of the catheter 210 in the desired location, at which point the clinician may once again release the control handle 300 to free up the use of both hands.

In step 522, as the distal portion 210a of the catheter 210 is further advanced within the body cavity of the patient, the fiber bragg grating 214 senses or otherwise measures contact pressure between the distal portion 210a of the catheter 210 and an interior portion of the body cavity of the patient. In step 524, the signal generated by the fiber bragg grating 214 is interpreted and haptic feedback is generated within the control handle 300 to mimic the pressure a user feels as the catheter 210 is advanced within the body cavity of the patient or when the catheter 210 is causing the body cavity of the patient to dilate. In step 526, a tool is inserted into the aperture 208 of the catheter housing 202 and advanced within a working channel of the catheter 210 until the tool is navigated to a surgical site. At this point, the tool may be utilized to take a biopsy, resect tissue, etc.

Although described generally hereinabove, it is envisioned that the memory 32 may include any non-transitory computer-readable storage media for storing data and/or software including instructions that are executable by the processor 30 and which control the operation of the workstation 20 and, in some embodiments, may also control the operation of the endoscope 100 and/or robotic surgical system 50. In an embodiment, the memory 32 may include one or more storage devices such as solid-state storage devices, e.g., flash memory chips. Alternatively, or in addition to the one or more solid-state storage devices, the memory 32 may include one or more mass storage devices connected to the processor 30 through a mass storage controller (not shown) and a communications bus (not shown).

Although the description of computer-readable media contained herein refers to solid-state storage, it should be appreciated by those skilled in the art that computer-readable storage media can be any available media that can be accessed by the processor 30. That is, computer readable storage media may include non-transitory, volatile and non-volatile, removable and non-removable media implemented in any method or technology for storage of information such as computer-readable instructions, data structures, program modules or other data. For example, computer-readable storage media may include RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which may be used to store the desired information, and which may be accessed by the workstation 20.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of embodiments. Those skilled in the art will envision other modifications within the scope and spirit of the claims appended hereto.

The invention may be described by reference to the following numbered paragraphs:-
1. A system for performing a surgical procedure, comprising:
   an endoscope, the endoscope comprising:
   a catheter attachment; and
   a control handle, the control handle selectively coupled to a portion of the catheter attachment such that inputs received by the control handle effectuate corresponding reactions of a portion of the catheter attachment when the control handle is coupled to the catheter attachment and when the control handle is separated from the catheter attachment.
2. The system according to paragraph 1, wherein the catheter attachment includes a catheter housing and a catheter operably coupled to, and extending distally from, a portion of the catheter housing.
3. The system according to paragraph 2, wherein the catheter housing includes a drive mechanism, the drive mechanism disposed within a portion of the catheter housing and configured to effectuate articulation of a distal portion of the catheter.
4. The system according to paragraph 3, wherein the drive mechanism includes a motor, wherein the motor causes the drive mechanism to effectuate articulation of the distal portion of the catheter.
5. The system according to paragraph 3, wherein the control handle includes a motor disposed therein, wherein the motor is operably coupled to the drive mechanism when the catheter housing is coupled to the control handle.
6. The system according to paragraph 2, further comprising a robotic surgical system, a portion of the robotic surgical system configured to be selectively coupled to a portion of the catheter housing when the catheter housing is separated from the control handle.
7. The system according to paragraph 6 wherein the catheter housing includes a drive mechanism disposed therein, the drive mechanism selectively couplable to a portion of the robotic surgical system such that when the catheter housing is coupled to the robotic surgical system, the robotic surgical system is operably coupled to the drive mechanism.
8. The system according to paragraph 7, wherein the control handle is in selective communication with the robotic surgical system, wherein inputs received on the control handle are received by the robotic surgical system to cause the robotic surgical system to effectuate a corresponding reaction of the catheter.
9. The system according to paragraph 8, wherein the control handle further includes an inertial measurement unit, wherein movement of the control handle is measured by the inertial measurement unit to effectuate a corresponding reaction of the catheter.
10. The system according to paragraph 8, wherein the control handle includes a control pad, wherein inputs received on the control pad cause the robotic surgical system to effectuate a corresponding reaction of the catheter.
11. A method for performing a surgical procedure, comprising:
   navigating a distal portion of a catheter attachment within a body cavity of a patient using a control handle selectively coupled to a portion of the catheter attachment;
   separating the control handle from the catheter attachment;
   coupling the catheter attachment to a portion of a robotic surgical system; and
   navigating the distal portion of the catheter attachment to a surgical site using the control handle to remotely control movement of the catheter attachment via the robotic surgical system.
12. The method according to paragraph 11, wherein navigating a distal portion of the catheter attachment includes articulating a distal portion of a catheter operably coupled to a portion of the catheter attachment using a drive mechanism disposed within a portion of the catheter attachment.
13. The method according to paragraph 12, wherein navigating a distal portion of the catheter includes articulating the distal portion of the catheter using a motor operably coupled to the drive mechanism and disposed within a portion of the catheter attachment.
14. The method according to paragraph 12, wherein navigating a distal portion of the catheter includes articulating the distal portion of the catheter using a motor operably coupled to the drive mechanism and disposed within a portion of the control handle.
15. The method according to paragraph 14, wherein coupling the catheter attachment to a portion of the robotic surgical system includes coupling a motor disposed within a portion of the robotic surgical system to the drive mechanism of the catheter attachment.
16. A system for performing surgery, comprising:
   a catheter housing, the catheter housing including a catheter operably coupled to, and distally extending from, a portion of the catheter housing;
   a control handle, the control handle selectively coupled to a portion of the catheter housing and configured to receive inputs to effectuate reactions of the catheter of the catheter housing;
   a robotic surgical system, a portion of the robotic surgical system configured to be operably coupled to the catheter housing when the catheter housing is separated from the control handle.
17. The system according to paragraph 16, wherein the catheter housing includes a drive mechanism, the drive mechanism disposed within a portion of the catheter housing and configured to effectuate articulation of a distal portion of the catheter.
18. The system according to paragraph 17, wherein the control handle includes a motor, the motor disposed within a portion of the control handle and configured to be operably coupled to the drive mechanism when the catheter housing is coupled to the control handle.
19. The system according to paragraph 17, wherein the robotic surgical system includes a motor, the motor disposed within a portion of the robotic surgical system and configured to be operably coupled to the drive mechanism when the catheter housing is coupled to the robotic surgical system.
20. The system according to paragraph 19, wherein the control handle is configured to directly control articulation of the distal portion of the catheter when the catheter housing is coupled to the control handle and is configured to remotely control articulation of the distal portion of the catheter when the catheter housing is coupled to the robotic surgical system.

## Claims

1. A system for performing a surgical procedure, comprising:
an endoscope, the endoscope comprising:
a catheter attachment; and
a control handle, the control handle selectively coupled to a portion of the catheter attachment such that inputs received by the control handle effectuate corresponding reactions of a portion of the catheter attachment when the control handle is coupled to the catheter attachment and when the control handle is separated from the catheter attachment.

2. The system according to claim 1, wherein the catheter attachment includes a catheter housing and a catheter operably coupled to, and extending distally from, a portion of the catheter housing.

3. The system according to claim 2, wherein the catheter housing includes a drive mechanism, the drive mechanism disposed within a portion of the catheter housing and configured to effectuate articulation of a distal portion of the catheter; preferably wherein the drive mechanism includes a motor, wherein the motor causes the drive mechanism to effectuate articulation of the distal portion of the catheter.

4. The system according to claim 3, wherein the control handle includes a motor disposed therein, wherein the motor is operably coupled to the drive mechanism when the catheter housing is coupled to the control handle.

5. The system according to claim 2, further comprising a robotic surgical system, a portion of the robotic surgical system configured to be selectively coupled to a portion of the catheter housing when the catheter housing is separated from the control handle; preferably wherein the catheter housing includes a drive mechanism disposed therein, the drive mechanism selectively couplable to a portion of the robotic surgical system such that when the catheter housing is coupled to the robotic surgical system, the robotic surgical system is operably coupled to the drive mechanism.

6. The system according to claim 5, wherein the control handle is in selective communication with the robotic surgical system, wherein inputs received on the control handle are received by the robotic surgical system to cause the robotic surgical system to effectuate a corresponding reaction of the catheter; preferably wherein the control handle further includes an inertial measurement unit, wherein movement of the control handle is measured by the inertial measurement unit to effectuate a corresponding reaction of the catheter.

7. The system according to claim 6, wherein the control handle includes a control pad, wherein inputs received on the control pad cause the robotic surgical system to effectuate a corresponding reaction of the catheter.

8. A system for performing surgery, comprising:
a catheter housing, the catheter housing including a catheter operably coupled to, and distally extending from, a portion of the catheter housing;
a control handle, the control handle selectively coupled to a portion of the catheter housing and configured to receive inputs to effectuate reactions of the catheter of the catheter housing;
a robotic surgical system, a portion of the robotic surgical system configured to be operably coupled to the catheter housing when the catheter housing is separated from the control handle.

9. The system according to claim 8, wherein the catheter housing includes a drive mechanism, the drive mechanism disposed within a portion of the catheter housing and configured to effectuate articulation of a distal portion of the catheter.

10. The system according to claim 9, wherein the control handle includes a motor, the motor disposed within a portion of the control handle and configured to be operably coupled to the drive mechanism when the catheter housing is coupled to the control handle.

11. The system according to claim 10, wherein the robotic surgical system includes a motor, the motor disposed within a portion of the robotic surgical system and configured to be operably coupled to the drive mechanism when the catheter housing is coupled to the robotic surgical system.

12. The system according to claim 11, wherein the control handle is configured to directly control articulation of the distal portion of the catheter when the catheter housing is coupled to the control handle and is configured to remotely control articulation of the distal portion of the catheter when the catheter housing is coupled to the robotic surgical system.
